# EUROPEAN PATENT SPECIFICATION

(11) **EP 4 236 911 B1**
(45) Date of publication and mention of the grant of the patent: **18.02.2026**
(21) Application number: 21782573.6
(22) Date of filing: 05.10.2021
(51) Int. Cl.: A61K 8/35, A61K 8/49, A61K 8/891, A61Q 5/00

(54) **HAIR CARE COMPOSITION**
HAARPFLEGEZUSAMMENSETZUNG
COMPOSITION DE SOINS CAPILLAIRES

(30) Priority: 27.10.2020 WO PCT/CN2020/124148; 15.12.2020 EP 20213967
(43) Date of publication of application: 06.09.2023
(73) Proprietor: Unilever IP Holdings B.V., 6708 WH Wageningen (NL); Unilever Global IP Limited, Wirral, Merseyside CH62 4ZD (GB)
(72) Inventor: CAO, Feng, Shanghai, Changning District 200335 (CN); CHEN, Guoqiang, Shanghai, Changning District 200335 (CN); JI, Chengdong, Shanghai, Changning District 200335 (CN); YUAN, Su, Shanghai, Putuo District 200000 (CN)
(74) Representative: Tansley, Sally Elizabeth
(86) International application number: PCT/EP2021/077433
(87) International publication number: WO 2022/089892

(56) References cited:
- DATABASE GNPD [online] MINTEL; 5 April 2016 (2016-04-05), ANONYMOUS: "Anti-Dandruff Shampoo", XP055809626, retrieved from https://www.gnpd.com/sinatra/recordpage/3900813/ Database accession no. 3900813
- DATABASE GNPD [online] MINTEL; 13 September 2018 (2018-09-13), ANONYMOUS: "Anti-Dandruff Shampoo", XP055809628, retrieved from https://www.gnpd.com/sinatra/recordpage/5950897/ Database accession no. 5950897
- DATABASE GNPD [online] MINTEL; 5 April 2016 (2016-04-05), ANONYMOUS: "Anti-Dandruff Shampoo", XP055809626, retrieved from https://www.gnpd.com/sinatra/recordpage/3900813/ Database accession no. 3900813
- DATABASE GNPD [online] MINTEL; 13 September 2018 (2018-09-13), ANONYMOUS: "Anti-Dandruff Shampoo", XP055809628, retrieved from https://www.gnpd.com/sinatra/recordpage/5950897/ Database accession no. 5950897

## Description

### Field of the Invention

The present invention relates to hair care compositions. More particularly the invention relates to hair care compositions that comprise piroctone olamine.

### Background of the Invention

Dandruff is a condition experienced by many people worldwide. The dandruff condition varies from mild symptoms such as flaking skin to severe inflammation and itchiness of the scalp. Malassezia yeasts, such as *Malassezia furfur,* are believed by some to be the main cause of dandruff and, whilst this might not represent the full scientific picture of the situation, Malassezia yeasts do appear to be closely associated with dandruff. Hence, the strategy conventionally used for the treatment of dandruff is the topical application of antifungals such as zinc pyrithione (ZnPTO), piroctone olamine, climbazole and ketoconazole which are normally delivered through a shampoo.

Piroctone olamine (also known as Octopirox^{®}) is a compound often used in the treatment of fungal infections. Piroctone olamine is the ethanolamine salt of the hydroxamic acid derivative piroctone. Piroctone olamine has anti-fungal properties that make it ideal for controlling the root cause of dandruff, a commonly occurring fungus called Malassezia globosa. It is often used in anti-dandruff shampoo as a replacement for the commonly used compound zinc pyrithione. Generally, piroctone olamine is dissolved in the base composition of a shampoo which includes surfactants, water, silicones, polymers and colouring and fragrance ingredients. When a user applies the compositions to hair and scalp, some compound of piroctone olamine get deposited thereon.

Generally, efficacy of an antidandruff shampoo or conditioner depends on the amount of antidandruff active deposited on the scalp and hair because more deposition means more bioavailability. However, it is well known that piroctone olamine is light instable and the piroctone olamine that is deposited is not bioavailable as piroctone olamine tends to react with UV radiation which leads to its destabilization. This destabilization can be avoided to some extent by using UV photostabilisers or sunscreens. Efforts have also been made to increase the antimicrobial efficacy of piroctone olamine by combining it with "booster" technologies.

### Summary of the Invention

The present inventors have determined a new way of improving the deposition of antidandruff agent such as piroctone olamine and meanwhile improve the photo stability of piroctone olamine. We have determined that both deposition and photo stability of piroctone olamine can be improved if piroctone olamine, a silicone compound and a specific benzophenone or benzophenone derivative are co-formulated into a composition.

In accordance with a first aspect, disclosed is a hair care composition comprising:
(i) a benzophenone derivative selected from benzophenone-2, benzophenone-3, and benzophenone-8 thereof;
(ii) 0.1-2% piroctone olamine by weight of the composition;
(iii) 0.5-3% silicone compound which is dimethicone, dimethiconol or a mixture thereof by weight of the composition; and
(iv) a cosmetically acceptable carrier;
wherein amount of said benzophenone derivatives is 0.2 to 10% by weight of the total composition.

In a second aspect, the present invention is directed to a method of preparing a hair care composition of any embodiment of the first aspect of this invention comprising the steps of:
(a) mixing said benzophenone derivative with said silicone compound to form a premix;
(b) mixing the premix of step (a) with other ingredients to form the hair care composition.

In accordance with a third aspect, disclosed is a method of treating a dandruff comprising a step of applying thereon a composition of the first aspect.

### Detailed Description of the Invention

For the avoidance of doubt, any feature of one aspect of the present invention may be utilized in any other aspect of the invention. The word "comprising" is intended to mean "including" but not necessarily "consisting of or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated. As used herein, the indefinite article "a" or "an" and its corresponding definite article "the" means at least one, or one or more, unless specified otherwise. The various features of the present invention referred to in individual sections above apply, as appropriate, to other sections *mutatis mutandis.* Consequently, features specified in one section may be combined with features specified in other sections as appropriate. Any section headings are added for convenience only, and are not intended to limit the disclosure in any way.

By "hair care composition" as used herein, is meant to include a composition for topical application to hair or scalp of mammals, especially humans. By topical is meant that the composition is applied to the external surface of the body. In the present invention this is achieved by applying the composition on the hair or scalp. Such a composition may be generally classified as leave-on or rinse off, and includes any product applied for improving the appearance, cleansing, odor control or general aesthetics of scalp and hair. The haircare composition of the present invention could be in the form of a liquid, lotion, cream, foam, scrub, gel, shampoo, conditioner, shower gel or bar. The haircare composition of the present invention is preferably a leave-on composition. Alternatively, the haircare composition of the present invention is a wash-off composition. Compositions for achieving the desired benefits by way of ingestion into the human body are excluded from the scope of the present invention.

### Benzophenone or benzophenone derivative

The composition in accordance with this invention comprises a benzophenone derivative selected from benzophenone-2, benzophenone- 3 and benzophenone-8 thereof, furthermore preferably selected from benzophenone-2 and benzophenone-3, most preferably is benzophenone-3.

### LogP

The partition coefficient (logP) describes the propensity of a compound to dissolve in an immiscible biphasic system of lipid (fats, oils, organic solvents) and water. A low value for logP means the compound has a higher affinity for the aqueous phase (it is more hydrophilic); a high value for logP denotes a higher concentration in the organic phase (i.e., the compound is more lipophilic).

The logP value is a constant defined in the following manner: LogP = log10 (Partition Coefficient) Partition Coefficient, P = [organic]/[aqueous]; where [ ] indicates the concentration of solute in the organic and aqueous partitions.

The log P of benzophenone, benzophenone-1, benzophenone-2, benzophenone-3, benzophenone-6, benzophenone-7, benzophenone-8, benzophenone-10 and benzophenone-12 is 3.21, 3.15, 3.09, 3.99, 4.78, 4.51, 4.31, 4.66 and 7.56 respectively.

Without wishing to be bound by theory the present inventors believe that lipophilic benzophenone or benzophenone derivative can bind with piroctone olamine and a carrier, leading to the co-deposition of piroctone olamine with a carrier. On one hand, lipophilic benzophenone or benzophenone derivative can be easily dispersed in carriers, like dimethicone whose logP is 2.67. On the other hand, the special aromatic structure of benzophenone or benzophenone derivative can have interaction with piroctone olamine, improving the carrier capacity for piroctone olamine, thereby improving the deposition of piroctone olamine.

Amount of the benzophenone derivatives in the composition of the invention would depend on the type of the hair care composition and the amount of piroctone olamine used. The composition in accordance with the invention comprises 0.2 to 10 wt% of said benzophenone or benzophenone derivatives, preferably 0.2 to 5 wt%, more preferably 0.3 to 2 wt%, furthermore preferably 0.4 to 1 wt% by weight of the composition.

### Piroctone olamine

Piroctone Olamine is an olamine salt of the hydroxamic acid derivative piroctone which is a typical antimicrobial active. It is commonly known as piroctone ethanolamine with the trade name Octopirox^{®}.

The piroctone olamine according to the present invention is a 1:1 compound of 1-hydroxy-4-methyl-6-(2,4,4-trimethylpentyl)-2(*1H*)-pyridinone with 2-aminoethanol and is also designated 1-hydroxy-4-methyl-6-(2,4,4-trimethylpentyl)-2(*1H*) pyridinone monoethanolamine salt. The CAS number is 68890-66-4 and the compound has the general formula as below:

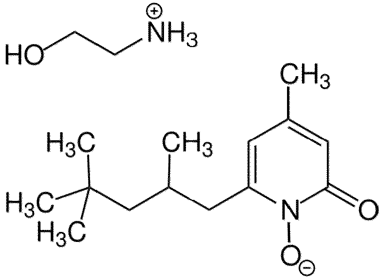

Several antidandruff hair care products contain piroctone olamine. It is found that dissolved intact piroctone olamine molecules are the sole bioactive form of piroctone olamine. When a hair care product with piroctone olamine is utilized, the piroctone olamine dissociates and undergoes photo-oxidation due to which stability of the bioactive is affected which reduces its antidandruff efficacy.

Amount of the piroctone olamine in the composition of the invention would depend on the type of the hair care composition and the precise nature of other antidandruff agents used. The composition comprises 0.1 to 2 wt% by weight of the composition.

The the weight ratio of benzophenone derivatives to the piroctone olamine is preferably 1:0.1 to 1:10, more preferably 1:0.5 to 1:5, furthermore preferably 1:1 to 1:3.

### Silicone

The hair care composition of the invention comprises a silicone compound.

Suitable silicones include polydiorganosiloxanes, polydimethylsiloxanes which have the CTFA designation dimethicone. In addition, suitable for use in the compositions of the invention (particularly shampoos and conditioners) are polydimethyl siloxanes having hydroxyl end groups, which have the CTFA designation dimethiconol.

Preferably the viscosity of the emulsified silicone is at least 10,000 cst at 25 °C, the viscosity of the silicone is preferably at least 60,000 cst, most preferably at least 500,000 cst, ideally at least 1,000,000 cst. Preferably the viscosity does not exceed 10⁹ cst for ease of formulation (1 cSt=1 mm².s⁻¹).

Examples of suitable pre-formed emulsions include Xiameter MEM 1785 and microemulsion DC2-1865 available from Dow Corning. These are emulsions /microemulsions of dimethiconol.

The total amount of silicone is 0.5 to 3 wt%.

### Hair care composition

In accordance with a further aspect of the invention is disclosed a hair care composition of the first aspect. Preferably the composition is a shampoo, hair conditioner, hair cream, hair gel, hair serum, mousse or hair oil. More preferably the composition is a shampoo composition.

The composition of the invention especially shampoos are formulated preferably with an anionic surfactant e.g. an alkyl sulphate and/or alkoxylated alkyl sulfate surfactant. These anionic surfactants are preferably present at a level of from 2 to 16%, more preferably from 3 to 16% by weight of the composition. Preferred alkyl sulfates are C8-18 alky sulfates, more preferably C12-18 alkyl sulfates, preferably in the form of a salt with a solubilising cation such as sodium, potassium, ammonium or substituted ammonium. Examples are sodium lauryl sulfate (SLS) or sodium dodecyl sulfate (SDS).

Preferred alkyl ether sulfates are those having the formula: RO (CH2CH2O) nSO¬3M; wherein R is an alkyl or alkenyl having from 8 to 18 (preferably 12 to 18) carbon atoms; n is a number having an average value of greater than at least 0.5, preferably between 1 and 3, more preferably between 1 and 2; and M is a solubilising cation such as sodium, potassium, ammonium or substituted ammonium. An example is sodium lauryl ether sulfate (SLES).

Preferred alkoxylated alkyl sulfate anionic surfactant is sodium lauryl ether sulfate (SLES) having an average degree of ethoxylation of from 0.5 to 3, preferably 1 to 3.

The composition as per the invention optionally and preferably additionally comprises a betaine surfactant. In a preferred embodiment, the composition comprises from 0.1 to 10 wt.%, preferably from 0.5 to 8 wt.%, more preferably from 1 to 5 wt.% of a betaine surfactant, preferably an alkyl amidopropyl betaine, for example cocamidopropyl betaine.

Preferably, the hair care composition of the invention in the form of a shampoo comprise a surfactant which is sodium lauryl sulphate, sodium lauryl ether sulphate, sodium lauryl ether sulphosuccinate, ammonium lauryl sulphate, ammonium lauryl ether sulphate, sodium cocoyl isethionate and lauryl ether carboxylic acid, coco betaine, cocamidopropyl betaine, sodium cocoamphoacetate or a mixture thereof.

Preferably, the hair care composition of the present invention comprises from 1 to 50%, preferably from 2 to 40%, more preferably from 4 to 25% total surfactant.

The hair conditioning composition comprises conditioning surfactants selected from cationic surfactants, used singly or in admixture. Preferably, the cationic surfactants have the formula N+R1R2R3R4 wherein R1, R2, R3 and R4 are independently (C1 to C30) alkyl or benzyl. Preferably, one, two or three of R1, R2, R3 and R4 are independently (C4 to C30) alkyl and the other R1, R2, R3 and R4 group or groups are (C1-C6) alkyl or benzyl. More preferably, one or two of R1, R2, R3 and R4 are independently (C6 to C30) alkyl and the other R1, R2, R3 and R4 groups are (C1-C6) alkyl or benzyl groups. Optionally, the alkyl groups may comprise one or more ester (-OCO- or -COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (eg, oleyl). Alkyl groups are optionally ethoxylated on the alkyl chain with one or more ethyleneoxy groups.

Suitable cationic surfactants for use in conditioner compositions according to the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (eg, Arquad 2HT/75 from Akzo Nobel), cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in conditioners according to the invention is cetyltrimethylammonium chloride, available commercially, for example as GENAMIN CTAC, ex Hoechst Celanese. Another particularly useful cationic surfactant for use in conditioners according to the invention is behenyltrimethylammonium chloride, available commercially, for example as GENAMIN KDMP, ex Clariant. Yet another preferred cationic surfactant is stearamidopropyl dimethylamine.

The most preferred cationic surfactants for use in the composition are stearamidopropyl dimethylamine, behentrimonium chloride, or stearyl trimethyl ammonium chloride. In conditioners of the invention, the level of cationic surfactant will generally range from 0.1 to 5%, preferably 0.5 to 2.5% by weight of the composition.

Hair conditioning compositions of the invention preferably may also additionally comprise a fatty alcohol. The combined use of fatty alcohols and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is also advantageous in that they contribute to the overall conditioning properties of compositions of the invention.

The level of fatty alcohol in conditioners of the invention will generally range from 0.5 to 10%, preferably from 0.1 to 8%, more preferably from 0.2 to 7 %, most preferably from 0.3 to 6 % by weight of the composition. The weight ratio of cationic surfactant to fatty alcohol is suitably from 1:1 to 1:10, more preferably from 1:1.5 to 1:8, optimally from 1:2 to 1:5.

It is further preferred that the hair care composition of the invention comprises a cosmetic ingredient. Preferably the cosmetic ingredient is selected from the group consisting of a silicone, an antibacterial agent other than antidandruff agents, a foam booster, a perfume, encapsulates (for example encapsulated fragrance) a dye, a colouring agent, a pigment, a preservative, a thickener, a protein, a phosphate ester, a buffering agent, a pH adjusting agent, a pearlescer (for example; mica, titanium dioxide, titanium dioxide coated mica, ethylene glycol distearate (INCl glycol distearate)) and/or opacifier, a viscosity modifier, an emollient, a sunscreen, an emulsifier, a sensate active (for example menthol and menthol derivatives), vitamins, mineral oils, essential oils, lipids, natural actives, glycerin, natural hair nutrients such as botanical extracts, fruit extracts, sugar derivatives and amino acids, microcrystalline cellulose and mixtures thereof.

Preferably, the hair care composition of the present invention includes from 0.01 to 20 wt% of the at least one cosmetic ingredient, more preferably from 0.05 to 10 wt%, still more preferably from 0.075 to 7.5 wt% and most preferably, from 0.1 to 5 wt% of the at least one cosmetic ingredient, by weight of the total composition.

The hair care composition of the present invention may also comprise synergistic antimicrobial compounds that give synergistic antimicrobial benefit when used in combination with the piroctone olamine to enhance its properties and further inhibit the growth of Malassezia furfur. Non-limiting examples of these compounds include compounds having alcoholic groups (e.g. honokiol, magnolol or paeonol), Piperazines and a phenolic compound found in natural plant extract viz. thymol and terpeniol.

The composition may additionally comprise a vitamin B3 compound. The preferred vitamin B3 compound is niacinamide.

Niacinamide is known for secretion of AMPs (Anti-Microbial Proteins) from keratinocytes. The AMPs thus secreted provides for improving immunity of e.g. the scalp. Thus with the use of niacinamide, the anti-dandruff efficacy can be enhanced not just through anti-fungal activity but by boosting the scalp's own protection shield against germs, through use of niacinamide. This combination could provide further long-lasting protection e.g. up to 24 hours of protection against germs.

When present, it is preferred that the hair care composition of the invention comprises 0.1 to 5% niacinamide, more preferably 0.5 to 5%, furthermore preferably 0.5 to 3%, and optimally 1.0 to 3.0% by weight of the composition.

### Shampoos

When the haircare composition of the invention is a shampoo, it is generally aqueous, i.e. they have water or an aqueous solution or a lyotropic liquid crystalline phase as their major component.

Suitably, the shampoo composition comprises 50 to 98%, preferably from 60 to 92% water.

Preferably the shampoo composition comprises one or more cationic polymers for conditioning the hair.

Suitable cationic polymers include homopolymers which are cationically substituted or may be formed from two or more types of monomers. The weight average (M_{w}) molecular weight of the polymers will generally be between 100000 and 3 Million Daltons. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus, when the polymer is not a homopolymer it can contain spacer non-cationic monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/g. The cationic charge density of the polymer is suitably determined via the Kjeldahl method as described in the US Pharmacopoeia under chemical tests for nitrogen determination.

Suitable cationic polymers include copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general, secondary and tertiary amines, especially tertiary, are preferred.

Amine substituted vinyl monomers and amines can be polymerised in the amine form and then converted to ammonium by quaternization.

The cationic polymers can comprise mixtures of monomer units derived from amine- and/or quaternary ammonium-substituted monomer and/or compatible spacer monomers.

Suitable (non-limiting examples of) cationic polymers include:
- cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and co-polymers of unsaturated carboxylic acids having from 3 to 5 carbon atoms, (as described in U.S. Patent 4,009,256);
- cationic polyacrylamides (as described in WO95/22311).

Other cationic polymers that can be used include cationic polysaccharide polymers, such as cationic cellulose derivatives, cationic starch derivatives, and cationic guar gum derivatives.

A particularly suitable type of cationic polysaccharide polymer that can be used is a cationic guar gum derivative, such as guar hydroxypropyltrimethylammonium chloride (commercially available from Rhodia in their JAGUAR trademark series). Examples of such materials are JAGUAR C13S, JAGUAR C14 and JAGUAR C17.

Mixtures of any of the above cationic polymers may be used.

It is preferred that the hair care composition of the invention comprises 0.01 to 5%, preferably from 0.02 to 1%, more preferably from 0.05 to 0.8% cationic polymer.

The hair care composition of the invention may additionally comprise a cationic deposition polymer which is a cationic polygalactomannans having an average molecular weight (*M_{w}*) of from 1 million to 2.2 million g/mol and a cationic degree of substitution of from 0.13 to 0.3.

The polygalactomannans are polysaccharides composed principally of galactose and mannose units and are usually found in the endosperm material of seeds from leguminous plants such as guar, locust bean, honey locust, flame tree, and other members of the *Leguminosae* family. Polygalactomannans are composed of a backbone of 1→4-linked β-D-mannopyranosyl main chain units (also termed mannoside units or residues) with recurring 1→6-linked α-D-galactosyl side groups (also termed galactoside units or residues) branching from the number 6 carbon atom of a mannopyranose residue in the polymer backbone. The polygalactomannans of the different *Leguminosae* species differ from one another in the frequency of the occurrence of the galactoside side units branching from the polymannoside backbone. The mannoside and galactoside units are generically referred to herein as glycoside units or residues. The average ratio of mannoside to galactoside units in the polygalactomannan contained in guar gum (hereinafter termed "guar") is approximately 2:1.

Suitable cationic polygalactomannans include guar and hydroxyalkyl guar (for example hydroxyethyl guar or hydroxypropyl guar), that has been cationically modified by chemical reaction with one or more derivatizing agents.

In a typical composition the amount of cationic polygalactomannans will generally range from about 0.05 to 1%, preferably from 0.1 to 0.8%, more preferably 0.2 to 0.6% by weight of the composition.

The hair care composition of the invention may additionally comprise an anionic polymeric rheology modifier such as a carboxylic acid polymer.

The term "carboxylic acid polymer" in the context of this invention generally denotes a homopolymer or copolymer obtained from the polymerization of ethylenically unsaturated monomers containing pendant carboxylic acid groups (hereinafter termed "carboxylic monomers").

Suitable carboxylic monomers generally have one or two carboxylic acid groups, one carbon to carbon double bond and contain a total of from 3 to about 10 carbon atoms, more preferably from 3 to about 5 carbon atoms.

Specific examples of suitable carboxylic monomers include α-β-unsaturated monocarboxylic acids such as acrylic acid, methacrylic acid and crotonic acid; and α-β-unsaturated dicarboxylic acids such as itaconic acid, fumaric acid, maleic acid and aconitic acid. Salts, esters or anhydrides of the α-β-unsaturated mono- or dicarboxylic acids described above may also be used. Examples include half esters of α-β-unsaturated dicarboxylic acids with C₁₋₄ alkanols, such as monomethyl fumarate; cyclic anhydrides of α-β-unsaturated dicarboxylic acids such as maleic anhydride, itaconic anhydride and citraconic anhydride; and esters of acrylic acid or methacrylic acid with C₁₋₃₀ alkanols, such as ethyl acrylate, butyl acrylate, 2-ethylhexyl acrylate, dodecyl acrylate, hexadecyl acrylate, and octadecyl acrylate.

Optionally, other ethylenically unsaturated monomers can be copolymerized into the carboxylic acid polymer backbone. Example of such other ethylenically unsaturated monomers include styrene, vinyl acetate, ethylene, butadiene, acrylonitrile and mixtures thereof. Carboxylic acid polymers may preferably have a molecular weight of at least 1 million Daltons.

Suitable examples include crosslinked copolymers polymerized from C₁₋₄ alkyl acrylate or methacrylate (e.g. ethyl acrylate) with one or more comonomers selected from acrylic acid, methacrylic acid and mixtures thereof. Such materials may generally be referred to under the INCl name of Acrylates Copolymer. Commercially available examples include Aculyn^{®} 33 from Rohm and Haas.

Also suitable are crosslinked copolymers polymerized from C₁₀₋₃₀ alkyl esters of acrylic or methacrylic acid with one or more comonomers selected from acrylic acid, methacrylic acid and their respective C₁₋₄ alkyl esters. Such materials may generally be referred to under the INCl name of Acrylates/C10-30 Alkyl Acrylate Crosspolymer. Commercially available examples include Carbopol^{®} polymers 1342 and 1382 from Lubrizol Advanced Materials.

Also suitable are optionally crosslinked copolymers of acrylic acid or methacrylic acid with alkyl acrylates and ethoxylated hydrophobically modified alkyl acrylates. Such materials may generally be referred to under the INCl names of Acrylates/Steareth-20 Methacrylate Copolymer, Acrylates/Beheneth-25 Methacrylate Copolymer, Acrylates/Steareth-20 Methacrylate Crosspolymer and Acrylates/Palmeth-25 Acrylates Copolymer. Commercially available examples include Aculyn^{®} 22, 28 or 88 from Rohm & Haas and Synthalen^{®} from 3V Sigma.

It is preferred that the carboxylic acid is a Carbomer, such as homopolymers of acrylic acid crosslinked with an allyl ether of pentaerythritol or an allyl ether of sucrose.

Mixtures of any of the aforementioned materials may also be used.

Preferably the hair care composition of the invention comprises 0.1 to 3.0%, more preferably 0.4 to 1.5% carboxylic acid polymer by weight of the composition.

In formulations containing anionic polymeric rheology modifiers such as the carboxylic acid polymers described above, it is often necessary to neutralize at least a portion of the free carboxyl groups by the addition of an inorganic or organic base. Examples of suitable inorganic or organic bases include alkali metal hydroxides (e.g. sodium or potassium hydroxide), sodium carbonate, ammonium hydroxide, methylamine, diethylamine, trimethylamine, monoethanolamine, triethanolamine and mixtures thereof.

The hair care composition of the invention may also comprise a nonionic polymeric rheology modifier which is selected from one or more nonionic cellulose ethers.

Suitable nonionic cellulose ethers or use as the nonionic polymeric rheology modifier in the invention include (C₁₋₃ alkyl) cellulose ethers, such as methyl cellulose and ethyl cellulose; hydroxy (C₁₋₃ alkyl) cellulose ethers, such as hydroxyethyl cellulose and hydroxypropyl cellulose; mixed hydroxy (C₁₋₃ alkyl) cellulose ethers, such as hydroxyethyl hydroxypropyl cellulose; and (C₁₋₃ alkyl) hydroxy (C₁₋₃ alkyl) cellulose ethers, such as hydroxyethyl methylcellulose and hydroxypropyl methylcellulose.

Preferred nonionic cellulose ethers for use as the nonionic polymeric rheology modifier in the invention are water-soluble nonionic cellulose ethers such as methylcellulose and hydroxypropyl methylcellulose. The term "water-soluble" in this context denotes a solubility in water of at least 1 gram, more preferably at least 3 grams, most preferably at least 5 grams in 100 grams of distilled water at 25° C. and 1 atmosphere. This level indicates production of a macroscopically isotropic or transparent, coloured or colourless solution.

Methyl cellulose and hydroxypropyl methylcellulose are commercially available in a number of viscosity grades from Dow Chemical as their METHOCEL^{®} trademark series.

Mixtures of any nonionic cellulose ethers may also be suitable. In a typical composition according to the invention the level of nonionic cellulose ethers will generally range from about 0.01 to about 2.0%, and preferably ranges from 0.1 to 0.5%, more preferably from 0.1 to 0.3%, by weight based on the total weight of the composition.

Preferably the haircare composition of the invention comprises 0.1 to 0.3% by weight nonionic cellulose ether.

The hair care composition of the invention may contain further optional ingredients to enhance performance and/or consumer acceptability. Examples of such ingredients include fragrance, dyes and pigments and preservatives. Each of these ingredients will be present in an amount effective to accomplish its purpose. Generally, these optional ingredients are included individually at a level of up to 5% by weight based on the total weight of the composition.

### Method of preparing hair care composition

This invention also relates to a method of preparing a hair care composition comprising the steps of:
(a) mixing said benzophenone or benzophenone derivative with said silicone compound to form a premix;
(b) mixing the premix of step (a) with other ingredients to form the hair care composition.

The benzophenone derivative is selected from benzophenone-2, benzophenone- 3 and benzophenone-8 thereof, most preferably is benzophenone-3.

The silicone compound is selected from dimethicone, dimethiconol or a mixture thereof.

### Method and Use

The present invention also provides for a method of treating a dandruff comprising a step of applying thereon a composition of the first aspect. The method is non-therapeutic in nature. Preferably it is for cosmetic purpose.

The present invention also provides for use of a composition of the first aspect to treat a dandruff.

### Mode of Use

The hair care composition of the invention is primarily intended for topical application to hair and scalp.

When the composition is a shampoo, it is topically applied to the hair and then massaged into the hair and scalp. Then it is rinsed with water prior to drying the hair. A hair oil or hair serum, being leave-on hair care compositions, are left on for 1 to 10 hours after application before being washed off.

The invention will be further illustrated by the following, non-limiting Examples, in which all percentages quoted are by weight based on total weight unless otherwise stated.

The examples are intended to illustrate the invention and are not intended to limit the invention to those examples *per se.*

### Examples

### Example 1: Octopirox^{®} deposition of the shampoo compositions with or without the combination of benzophenone or benzophenone derivative and silicone.

### Preparation of hair care compositions

The following shampoo compositions as shown in Table-1 were prepared:
When preparing composition 1, the benzophenone-3 and dimethicone are premixed before mixing with other ingredients to form the composition.

**Table-1**

| **Ingredients (wt%)** | **Compositions** | | | |
|---|---|---|---|---|
| | **A** | **B** | **C** | **1** |
| SLES | 13 | 13 | 13 | 13 |
| CAPB | 1.5 | 1.5 | 1.5 | 1.5 |
| Carbomer | 0.35 | 0.35 | 0.35 | 0.35 |
| Octopirox^{®} | 1 | 1 | 1 | 1 |
| Benzophenone-3 | - | 0.4 | - | 0.4 |
| Dimethicone | - | - | 1.5 | 1.5 |
| Wate | To 100 | To 100 | To 100 | To 100 |

### Octopirox^{®} deposition test method

Vitro-skin (supplied by IMS Inc) is used to mimic human scalp. The skin is cut into proper size and fastened with plastic ring. 0.20 g of the tested shampoo composition is added onto vitro-skin, followed by 1.8 mL deionized water. Stir and rub the shampoo onto the entire surface by stirring rod for 30s and take out all shampoo solution using dropper. Then rinse the vitro-skin with 4 mL of deionized water. Stir and rub the entire surface again for 30s. Take out all rinsed water and repeat rinsing for one more time. After the vitro-skin is completely dry, put the skin into centrifuge tube with 10 mL methanol, followed by supersonic extraction for 30 min. 4 mL methanol is transferred to a new centrifuge tube and evaporate the methanol completely by vacuum concentrator. Reconstitute with 0.4 mL methanol and transfer 0.2 mL sample into 2 mL ultra performance liquid chromatography (UPLC) brown vial with Micro-Inserts.

A Waters^{®} ACQUITY ultra performance liquid chromatography (UPLC, Waters, Manchester, UK) was used for the sample analysis. MassLynx software (version 4.1) was used for instrument control and data acquisition. Separation was carried out on a Waters Acquity UPLC BEH C18 column (2.1 × 100 mm, 1.7 µm particle size). The mobile phase A was composed of 0.1% formic acid + 5µM medronic acid in DI water and the mobile phase B was composed of 0.1% formic acid in acetonitrile, programmed in a gradient mode. The sample was measured by photo-diode array (PDA) detector at absorption wavelength of 302 nm.

The result of tested Octopirox^{®} deposition is showed in Table-2 below:

**Table-2**

| **Compositions** | **A** | **B** | **C** | **1** |
|---|---|---|---|---|
| Octopirox^{®} deposition (µg/piece) | 14.67 ± 0.80 | 12.78 ± 1.58 | 15.23 ± 1.02 | 18.91 ± 1.80 |

The results showed that composition 1 comprising a combination of benzophenone-3 and dimethicone provided significantly better deposition of piroctone olamine than compositions A (without benzophenone-3 and dimethicone), B (without dimethicone) and C (without benzophenone-3).

### Example 2: Impact of different benzophenone derivatives on the Octopirox^{®} deposition with UV exposure.

The following compositions as shown in Table-3 were prepared.

When preparing the composition D and 2, the benzophenone-3 or benzophenone-4 is premixed with dimethicone before mixing with other ingredients to form the composition.

**Table-3**

| **Ingredients (wt%)** | **Compositions** | |
|---|---|---|
| | **D** | **2** |
| SLES | 13 | 13 |
| CAPB | 1.5 | 1.5 |
| Carbomer | 0.35 | 0.35 |
| Octopirox^{®} | 1 | 1 |
| Benzophenone-3 (logP=3.99) | - | 0.4 |
| Benzophenone-4 (logP=0.99) | 0.4 | - |
| Dimethicone | 1.5 | 1.5 |
| Wate | To 100 | To 100 |

### Octopirox^{®} deposition test with UV exposure

Treat vitro-skin with shampoo compositions as protocol mentioned in the Example 1.

### UV treatment:

After the vitro-skin is completely dry, add 300 µL 50% water/50% methanol onto skin, followed by ultraviolet (UV) irradiation. The UV irradiation was carried out in an X-Rite (Macbeth) Spectra Light III chamber. UV mode was chosen for UV irradiation which provides both UVA and UVB lights. The UV light was tested by the UVX Radiometer (UVX-36, E24195, UVP.) and the intensity was estimated at 250 µw/cm² for UVA and 110 µw/cm² for UVB). The chamber temperature was equal to the room temperature (20 ± 2°C). The samples were placed in a line close to the center of the chamber for 4 h exposure. Dry the vitro-skin overnight and measure the Octopirox^{®} deposition with UPLC.

The deposited Octopirox^{®} was measured with UPLC as the method described in Example 1.

The result of tested Octopirox^{®} deposition is showed in Table-4 below:

**Table-4**

| **Compositions** | **D** | **2** |
|---|---|---|
| Octopirox^{®} deposition (µg/piece) | 16.93 ± 0.72 | 21.41 ± 1.00 |

The data in Table-4 clearly indicates that deposition of piroctone olamine with UV exposure is improved markedly when the composition in accordance with this invention is used (Composition 2) during the experiments, as compared to a corresponding example outside the invention (Composition D, not containing the benzophenone or benzophenone derivative of the present invention). The data further indicates that combination of benzophenone or benzophenone derivative of the present invention with silicone can improve the deposition and the photostability of piroctone olamine.

### Example 3: Impact of dosage of benzophenone derivatives on the Octopirox^{®} deposition with UV exposure.

The following compositions as shown in Table-5 were prepared.

When preparing the composition D and 2, the benzophenone-2 or benzophenone-3 is premixed with dimethicone before mixing with other ingredients to form the composition.

**Table-5**

| **Ingredients (wt%)** | **Compositions** | | | | | |
|---|---|---|---|---|---|---|
| | **E** | **F** | **G** | **3** | **4** | **5** |
| SLES | 13 | 13 | 13 | 13 | 13 | 13 |
| CAPB | 1.5 | 1.5 | 1.5 | 1.5 | 1.5 | 1.5 |
| Carbomer | 0.35 | 0.35 | 0.35 | 0.35 | 0.35 | 0.35 |
| Octopirox^{®} | 1 | 1 | 1 | 1 | 1 | 1 |
| Benzophenone-3 | - | - | 0.1 | 0.2 | 0.4 | - |
| Benzophenone-2 | - | - | - | - | - | 0.2 |
| Dimethicone | - | 1.5 | 1.5 | 1.5 | 1.5 | 1.5 |
| Water | To 100 | To 100 | To 100 | To 100 | To 100 | To 100 |

Octopirox^{®} deposition was tested by using the same method as disclosed in Example 1.

The result of tested Octopirox^{®} deposition is showed in Table-6 below:

**Table-6**

| **Compositions** | **E** | **F** | **G** | **3** | **4** | **5** |
|---|---|---|---|---|---|---|
| Octopirox^{®} deposition (µg/piece) | 22.84 ± 2.16 | 25.03 ± 0.92 | 23.94 ± 0.82 | 27.65 ± 1.56 | 31.45 ± 2.15 | 29.72 ± 2.00 |

The results showed that compositions 3-5 comprising a combination of benzophenone-3 or benzophenone-2 with a dosage of 0.2% or higher and dimethicone provided significantly better deposition of piroctone olamine than compositions E (without benzophenone derivative and dimethicone), F (without benzophenone derivative) and G (dosage of benzophenone derivative lower than 0.2%).

## Claims

1. A hair care composition comprising:
(i) a benzophenone derivative selected from benzophenone-2, benzophenone-3 and benzophenone-8;
(ii) 0.1 to 2% of a piroctone olamine by weight of the composition;
(iii) 0.5 to 3% of a silicone compound which is dimethicone, dimethiconol or a mixture thereof by weight of the composition; and
(iv) a cosmetically acceptable carrier;
wherein amount of said benzophenone derivative is 0.2 to 10% by weight of the total composition.

2. A hair care composition as claimed in claims 1 wherein amount of said benzophenone derivative is 0.3 to 5wt%.

3. A hair care composition as claimed in claims 1 or 2 additionally comprising a surfactant.

4. A hair care composition as claimed in claim 3 wherein the surfactant is an anionic surfactant or a cationic surfactant.

5. A hair care composition as claimed in claim 4 wherein the anionic surfactant is an alkyl sulphate and/or an alkoxylated alkyl sulfate surfactant.

6. A hair care composition as claimed in 5 wherein said composition comprises a betaine surfactant.

7. A hair care composition as claimed in any of claims 3 to 6 wherein the composition is a shampoo.

8. A hair care composition as claimed in claim 4 wherein the cationic surfactant is stearamidopropyl dimethylamine, behentrimonium chloride or stearyl trimethyl ammonium chloride.

9. A hair care composition as claimed in claim 8 wherein the composition is a hair conditioner.

10. A method of preparing a hair care composition as claimed in any of claims 1 to 9 comprising the steps of:
(a) mixing said benzophenone derivatives with said silicone compound to form a premix;
(b) mixing the premix of step (a) with other ingredients to form the hair care composition.

11. A composition as claimed in any of claims 1 to 9 for use in treating dandruff on a desired scalp surface.

## Patentansprüche

1. Haarpflegezusammensetzung, umfassend:
(i) ein Benzophenonderivat, ausgewählt unter Benzophenon-2, Benzophenon-3 und Benzophenon-8;
(ii) 0,1 bis 2% eines Piroctonolamins, bezogen auf das Gewicht der Zusammensetzung;
(iii) 0,5 bis 3% einer Silikonverbindung, die Dimethicon, Dimethiconol oder eine Mischung davon ist, bezogen auf das Gewicht der Zusammensetzung; und
(iv) einen kosmetisch akzeptablen Träger;
wobei die Menge des Benzophenonderivats 0,2 bis 10 Gew.-% der Gesamtzusammensetzung beträgt.

2. Haarpflegezusammensetzung nach Anspruch 1, wobei die Menge des Benzophenonderivats 0,3 bis 5 Gew.-% beträgt.

3. Haarpflegezusammensetzung nach Anspruch 1 oder 2, die zusätzlich ein Tensid enthält.

4. Haarpflegezusammensetzung nach Anspruch 3, wobei das Tensid ein anionisches Tensid oder ein kationisches Tensid ist.

5. Haarpflegezusammensetzung nach Anspruch 4, wobei das anionische Tensid ein Alkylsulfat- und/oder ein alkoxyliertes Alkylsulfat-Tensid ist.

6. Haarpflegezusammensetzung nach Anspruch 5, wobei die Zusammensetzung ein Betain-Tensid umfasst.

7. Haarpflegemittel nach einem der Ansprüche 3 bis 6, wobei das Mittel ein Shampoo ist.

8. Haarpflegezusammensetzung nach Anspruch 4, wobei das kationische Tensid Stearamidopropyldimethylamin, Behentrimoniumchlorid oder Stearyltrimethylammoniumchlorid ist.

9. Haarpflegezusammensetzung nach Anspruch 8, wobei die Zusammensetzung ein Haarconditioner ist.

10. Verfahren zur Herstellung einer Haarpflegezusammensetzung nach einem der Ansprüche 1 bis 9, umfassend die folgenden Schritte:
(a) Mischen der Benzophenonderivate mit der Silikonverbindung, um eine Vormischung zu bilden;
(b) Mischen der Vormischung aus Schritt (a) mit anderen Inhaltsstoffen, um die Haarpflegezusammensetzung zu bilden.

11. Zusammensetzung nach einem der Ansprüche 1 bis 9 zur Behandlung von Schuppen auf einer gewünschten Kopfhautoberfläche.

## Revendications

1. Composition capillaire comprenant:
(i) un dérivé de benzophénone choisi parmi la benzophénone-2, la benzophénone-3 et la benzophénone-8;
(ii) 0,1 à 2 % de piroctone olamine en poids de la composition;
(iii) 0,5 à 3 % d'un composé de silicone qui est la diméthicone, le diméthiconol ou un mélange de ceux-ci, en poids de la composition; et
(iv) un excipient cosmétiquement acceptable;
où la quantité dudit dérivé de benzophénone est 0,2 à 10 % en poids de la composition totale.

2. Composition capillaire selon la revendication 1, où la quantité dudit dérivé de benzophénone est 0,3 à 5 % en poids.

3. Composition capillaire selon les revendications 1 ou 2, comprenant en outre un tensioactif.

4. Composition capillaire selon la revendication 3, où le tensioactif est un tensioactif anionique ou un tensioactif cationique.

5. Composition capillaire selon la revendication 4, où le tensioactif anionique est un tensioactif alkylsulfate et/ou un tensioactif alkylsulfate alcoxylé.

6. Composition capillaire selon la revendication 5, où ladite composition comprend un tensioactif bétaïne.

7. Composition capillaire selon l'une quelconque des revendications 3 à 6, où la composition est un shampooing.

8. Composition capillaire selon la revendication 4, où le tensioactif cationique est la stéaramidopropyl diméthylamine, le chlorure de béhentrimonium ou le chlorure de stéaryl triméthyl ammonium.

9. Composition capillaire selon la revendication 8, où la composition est un après-shampooing.

10. Procédé de préparation d'une composition capillaire selon l'une quelconque des revendications 1 à 9, comprenant les étapes de:
(a) mélange desdits dérivés de benzophénone avec ledit composé de silicone pour former un prémélange;
(b) mélange du prémélange de l'étape (a) avec d'autres ingrédients pour former la composition capillaire.

11. Composition selon l'une quelconque des revendications 1 à 9, destinée à être utilisée dans le traitement des pellicules sur une surface du cuir chevelu souhaitée.
